# EUROPEAN PATENT SPECIFICATION

(11) **EP 0 504 255 B1**
(45) Date of publication and mention of the grant of the patent: **18.02.1998**
(21) Application number: 91901043.9
(22) Date of filing: 04.12.1990
(51) Int. Cl.: A61M 1/00, A61M 5/142

(54) **SELF-DRIVEN PUMP DEVICE**
SELBSTGESTEUERTE PUMPVORRICHTUNG
POMPE AUTOCOMMANDEE

(30) Priority: 05.12.1989 US 446182
(43) Date of publication of application: 23.09.1992
(73) Proprietor: McKinley, Inc., Ladewood, Colorado 80228 (US)
(72) Inventor: ZDEB, Brian, D., Round Lake Park, IL 60073 (US)
(74) Representative: Haecker, Walter
(86) International application number: US9007085
(87) International publication number: WO9108002

(56) References cited:
- WO-A-87/00758
- FR-A- 2 588 757
- US-A- 4 544 371
- US-A- 4 596 558
- US-A- 4 634 427
- US-A- 4 828 551

## Description

The present invention relates to an apparatus for the patient-controlled delivery of a beneficial agent according to the preamble of claim 1 for delivering a beneficial agent to a patient.

Specifically, the present invention is directed to the controlled delivery of a pre-selected quantity of beneficial agent to a patient, and is more particularly directed to an apparatus for such delivery wherein the amount of beneficial agent administered can be controlled, up to a maximum pre-selected amount.

An apparatus according to the preamble of claim 1 is known from US-A-4 544 371 which discloses an implantable drug delivery system which includes a domed body constructed of a resilient material that forms a dose reservoir for liquid medication which is to be injected into a patient. The patient receives a dosage by pressing upon the domed body, and the dose reservoir refills automatically after each dosage.

Another known apparatus of the claimed type which is disclosed in US-A-4 634 427 includes a domed body forming a dose reservoir for storing liquid medication. Compression of the domed body forces the liquid medication out of the domed body and into the patient. This known delivery assembly, however, requires a pressurized source of liquid medication in order to refill the dose reservoir.

Further, WO-A-8 700 758 discloses another apparatus and system for patient-controlled administration of an analgesic. This apparatus includes a dose reservoir for receiving and storing a dose of the analgesic, an inlet and an outlet to the dose reservoir, and control means operated by the patient for selectively expressing beneficial agent out of the dose reservoir through the outlet means. The inlet to the dose reservoir receives the analgesic under pressure from a pump means separate from the patient-controlled analgesic device itself which pumps the analgesic to the apparatus from an external supply source.

Generally, analgesics are often prescribed to relieve post-operative pain. The great difficulty in properly administering analgesics stems from a variety of factors. Age, hypatic function, renal function and other medication all affect the pharmacokinetics of analgesics and greatly affect the patient's need for analgesics. Thus while some patients never request (and thus do not need) analgesics for pain relief, some patients continue to suffer even after conventional doses of analgesics have been administered. Further, doctors tend to underprescribe the use of analgesics and nurses tend to underadminister them because of the fear that the patient will become addicted to the analgesic.

In the last several years, there has been considerable activity directed to devices and systems which permit the patient to control how much analgesic he or she receives up to a maximum predetermined limit. It has been found that as a group, patients controlling the quantity of analgesic they receive use less analgesic than patients who request the administration of an analgesic.

Apparently, one factor is the psychological relief present when a patient knows he or she is in control of the amount of drug to be received, up to a maximum limit.

Other devices that are on the market, or that are in the process of obtaining government regulatory approval, that are directed to the patient-controlled delivery of analgesics, include the Cardiff Palliator by Pye Dynamics Ltd. or Graseby Dynamics of the United Kingdom; the On-Demand Analgesic Computer (ODAC) Model JSI 0299 made by Janssen Scientific Instruments; a PCA infuser by Abbott Laboratories, Inc.; the Harvard PCA Pump by C.R. Bard Inc.; and a pump by Deltec Systems Inc. All of these pumps are large and bulky, the smallest pump being the Deltec pump, which is approximately as large as a telephone. All of the above-mentioned devices are electromechanical in nature, requiring a separate power source. Although the Deltec unit may conceivably be worn by patients, it is believed that the remainder of the pumps mentioned above confine the patient to a bed, or some other fixed location.

Another problem associated with these devices is that after the drug is loaded into the pump, certain control factors must be set by the nurse or other person who actually sets up the pump with the patient. Yet another problem with existing devices is that they are relatively expensive and may include some rather complex electronic components.

Known patient-controlled analgesic systems have several drawbacks. They are generally very complex and difficult to use. Often times they must be calibrated prior to use and this typically requires the user to be extensively trained in the use of the device. Also an auxiliary pumping source is typically needed to feed beneficial agent to the patient-controlled analgesic apparatus. In addition, such systems are comparatively expensive. Further, because of their size, many of the known patient-controlled analgesic systems are not suitable for ambulatory use. Thus there remains a need for an accurate, self-driven, low cost, patient-controlled analgesic apparatus, and in particular, such an apparatus that is adaptable for ambulatory use.

Accordingly it is a principal object of the present invention to provide a patient-controlled analgesic device which is self-driven for delivering a beneficial agent to a patient.

Regarding the apparatus the above object is accomplished by means of an apparatus comprising the features of claim 1.

It is an advantage of this invention that it provides a device which itself includes a single power source for both filling the device with beneficial agent and for discharging up to a maximum predetermined dose of the beneficial agent to the patient upon demand of the patient. It is a related advantage of this invention that it provides such a device wherein the power source has a linear fill rate over its entire fill range in order to prevent overdosing.

It is yet another advantage of the present invention that it provides a patient-controlled analgesic device that can be used with prefilled containers of the beneficial agent to be administered to the patient. It is also an advantage of the invention that it provides a patient-controlled analgesic device that is suitable for ambulatory use.

Generally speaking, the invention relates to a patient-controlled analgesic device capable of delivering a full dose or intermittently a fractional dose of a beneficial agent such as, for example, an analgesic, an antibiotic, heparin, insulin, or the like. The device includes a single power source comprising pump means which draws beneficial agent into the device at a relatively constant rate of flow and also serves as the means for delivering beneficial agent to the patient. To further control the rate at which beneficial agent is fed to the device, a restriction means (administration set) is used in combination with the patient-controlled analgesic device.

The apparatus of the present invention is intended to be used for delivering a beneficial agent to a patient by the steps of:
(a) compressing by means of an external force the collapsible dose reservoir illed with said agent to expel agent therefrom via outlet means into a conduit leading to the patient, said collapsible dose reservoir being capable of receiving and storing a dose of the beneficial agent and comprising inlet means to the dose reservoir for receiving the beneficial agent from an external supply, outlet means to the dose reservoir through which the beneficial agent in the dose reservoir exits the dose reservoir, and pump means operative in response to an external force supplied to said pump means for drawing beneficial agent into said dose reservoir at a relatively constant rate and for discharging the beneficial agent out of said dose reservoir through said outlet means, said pump means comprising an annular shaped arcuate wall means terminating at a foot, said arcuate wall means having a constant wall curvature and wall thickness extending from said foot to a centrally located head means, said head means having a rigidity substantially greater than the rigidity of the arcuate wall means to restrict the compression of said pump means substantially to said arcuate wall means, said pump means compressed in response to an external force applied to said pump means so as to advance the beneficial agent from said dose reservoir to said outlet means, and upon removal of the external force the pump means returns to the uncompressed condition at a relatively constant rate, wherein upon returning to the uncompressed condition the pressure in said dose reservoir is lower relative to the pressure in the external supply so that beneficial agent is drawn into said dose reservoir from the external supply via suction at a relatively constant rate; and of
(b) releasing said compression to allow said dose reservoir to refill with beneficial agent at a controlled, constant rate.

The above and other objects and advantages of the present invention will become apparent from the following detailed description of the invention and from the accompanying drawings, wherein:
FIGURE 1 is a perspective view of the patient-controlled analgesic device of the present invention and a supply source for beneficial agent;
FIGURE 2 is a top perspective view of the patient-controlled analgesic device and showing, in partial section the inlet and outlet means to the dose reservoir;
FIG. 3 is a cross-sectional view of the patient-controlled analgesic device taken along the line 3-3 in FIG. 2; and
FIG. 4 is a perspective view of the patient-controlled analgesic device mounted in an electronic unit capable of administering beneficial agent to the patient at pre-selected time intervals.

### DETAILED DESCRIPTION OF THE PREFERRED EMBODIMENTS

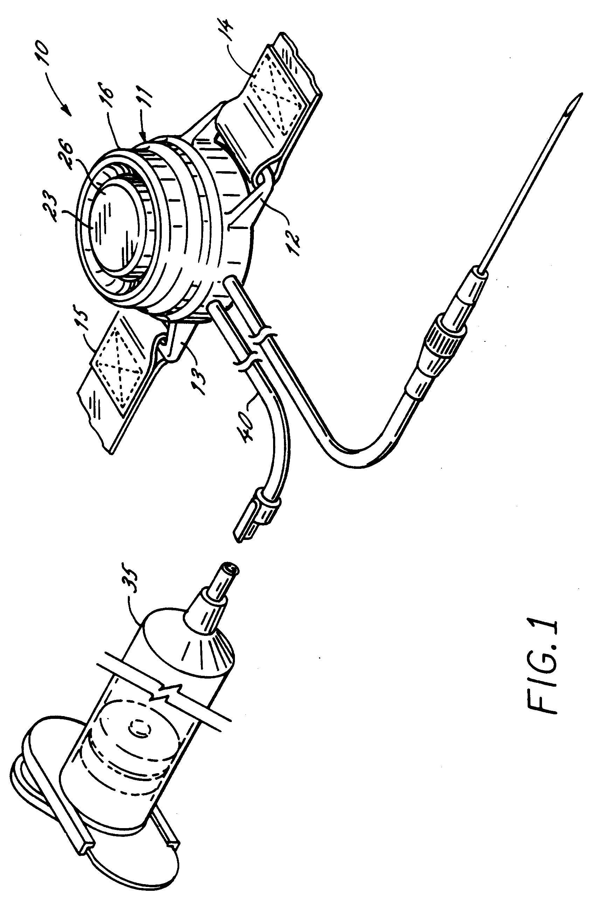

Referring now to FIGS. 1-3 the patient-controlled analgesic device 10 of the present invention includes a housing 11. Housing 11 forms mounting pins 12, 13 to which wrist band portions 14, 15 may be secured. The wrist band portions may be Velcro® or other bands that are capable of mating and securing the device to a wrist. The device 10 may thus be worn in the same manner as a watch.

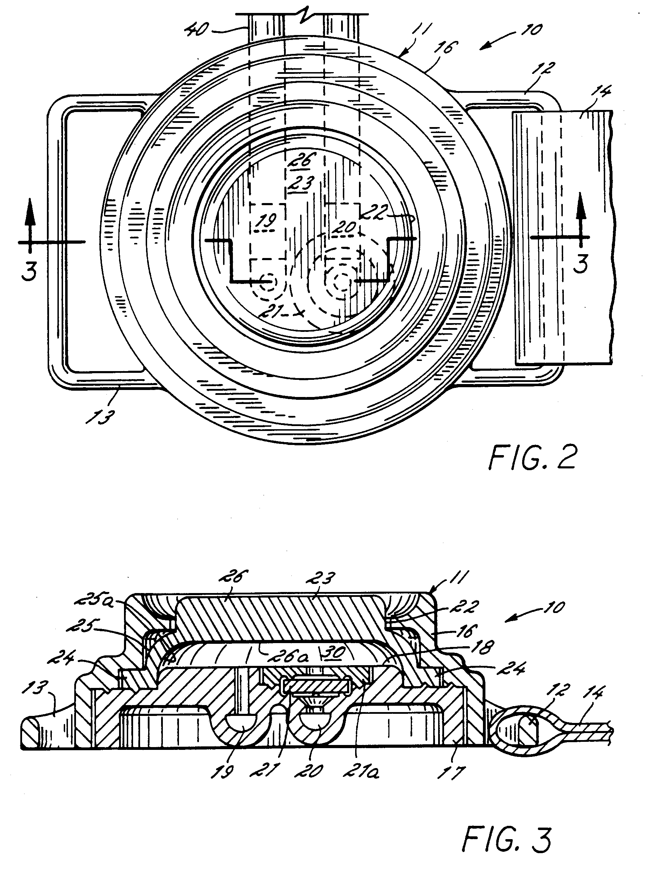

The housing 11 of the device 10 includes a casing 16 and a back plate 17 secured to the casing, as for example with a plurality of screws (not shown). A portion of the back plate 17 forms a raised plateau 18 that forms one wall of a dose reservoir 30. Back plate 17 further includes a dose reservoir inlet 19 in communication with the dose reservoir and a dose reservoir outlet 20 also in communication with the dose reservoir. Referring to FIG. 3, in the illustrated embodiment, the outlet 20 includes a disc valve 21, and valve seal 21a to sealingly engage disc valve 21 in communication with dose reservoir 30. Disc valve 21 is normally closed so that beneficial agent drawn into dose reservoir 30 is retained therein until expressed out of the device 10 by the patient, as will be more fully described below. It will be appreciated that the dose reservoir inlet 19 may include a valve means (not shown) or flow restrictive means (not shown) to limit backflow of beneficial agent through the inlet during operation of the device.

In the illustrated embodiment, pump means 23 has an annular cross section. Pump means 23 comprises a foot portion 24, an arcuate leg portion 25 and a head 26. The foot portion 24 of pump means 23 lies adjacent the plateau portion 18 of the back plate 17 and is in sealing engagement therewith. Arcuate-shaped leg portion 25 curves upwardly from foot portion 24 to head 26 of pump means 23. Head 26 of pump means 23 extends upwardly through annular opening 22 in casing 16 to facilitate access of the pump means to an external force, such as, for example, the patient. The interior portion 25a of arcuate-shaped leg portion 25 and the interior portion 26a of head 26 of pump means 23 together with the plateau 18 of back plate 17 define dose reservoir 30.

In accordance with one aspect of the present invention pump means 23 is designed so that it will draw beneficial liquid from a supply source 35, through conduit means 40 into dose reservoir 30 at a constant rate. It has been found that to accomplish that end, the arcuate-shaped portion 25 of pump means 23 should have a constant curvature over its entire length from foot portion 24 to head 26 and head 26 should be sufficiently rigid so that when pump means 23 is acted upon by an external force the arcuate-shaped leg portion 25 of pump means 23 will collapse under that force. The pump means so designed provides a substantially linear recovery rate throughout its entire range of motion relative to the dose reservoir. Stated another way, the rate at which the collapsed pump means recovers from a collapsed condition to its fully uncompressed starting position is linear over its entire stroke, from a fully compressed condition, as for example when the dose reservoir is completely emptied, to its original uncompressed condition, as for example when the dose reservoir is completely filled, and at all points between those two conditions.

The linear recovery rate of the pump means provides a constant suction to draw beneficial liquid from external supply source 35 into dose reservoir 30. Thus, after the pump means is depressed by an external force, such as the patient pushing on head 26 to discharge beneficial agent, upon release of the external force, head 26 returns to its original position at a controlled and linear rate. Accordingly, regardless of the position of the head relative to the dose reservoir after expressing beneficial agent from the dose reservoir by depressing the head, the incremental amount of beneficial agent drawn into the dose reservoir as the head rebounds to its original uncompressed position is directly proportional to the time the head was depressed by the external force. By way of example, if the recovery time to completely refill an emptied dose reservoir is T, then the recovery time will be one-half T for a dose reservoir that is only one-half emptied and one-fourth T for a dose reservoir that is only one-fourth emptied, et cetera.

The pump means is preferably constructed of a resilient material which is biocompatible with the human body and is likewise compatible with the beneficial agent to be administered to the patient. For example, silicone rubber is an acceptable resilient material. However, other elastomers can be used to construct the pump means.

In the illustrated embodiment of the invention shown in FIGS. 1-3, pump means 23 comprises a silicone rubber having a durometer of 30 to 50 (Shore A). A 30 to 50 durometer for the silicone elastomer pump means provides sufficient elasticity to perform the functions of both drawing beneficial agent from a supply source into the dose reservoir and expressing beneficial agent from the dose reservoir to the patient. It will be appreciated that a change in the durometer of the elastomer used for the pump means will affect the drawing power of the pump means and may thus affect the rate at which the dose reservoir is filled. For example, the greater the elastomer durometer, the greater the drawing power of the pump means will be.

As previously indicated, the head 26 of pump means 23 is more rigid than the arcuate-shaped leg portion 25. In the preferred embodiment, the pump means is a unitary structure so that head 26 must be thicker than the arcuate-shaped leg portion to impart the necessary rigidity to the head. Thus, head 26 must be sufficiently thick so that when external pressure is applied to it, pump means 23 is compressed by collapse of the arcuate-shaped leg portion 25. That is, the head 26 is sufficiently thick that it does not compress significantly under the externally-applied force. Further, as described, the arcuate-shaped leg portion 25 has a constant curvature over its entire length, and will collapse under the applicatioh of an external force in order to express beneficial agent out of the outlet means and to the patient.

In a preferred embodiment of the present invention, pump means 23 is formed from a silicone rubber elastomer with a durometer of 30 to 50 (Shore A), and the arcuate-shaped leg portion 25 has a thickness (in cross-section) of about two-thirds the thickness of head 26. It is especially preferred for such pump means that the head have a thickness of about 3 mm (0.12 inch) and the arcuate-shaped leg portion 25 have a width of about 2 mm (0.08 inch). The arcuate-shaped leg portion 25 has a uniform radius of curvature over its entire length, and together with head 26 define a pump means 23 having an annular area of constant cross-section.

As depicted in FIGS. 1-3, the resilient compressible pump means 23 assumes a first uncompressed condition. In this condition the patient-controlled apparatus may be primed for subsequent use by the patient by filling the dose reservoir with liquid and removing air from the apparatus.

As previously indicated, pump means 23 is operable in response to the application of an external force applied to head 26 of pump means 23. The external force may be, for example, the finger or thumb of the patient pushing downwardly on head 26. In response to the external force applied to head 26 of pump means 23, the arcuate-shaped leg portion 25 collapses. As the arcuate-shaped leg portion 25 of pump means 23 collapses, the normally closed valve means 21 in outlet means 20 opens and beneficial agent is expressed from the dose reservoir, through the outlet means to the patient. The valve means 21 remains open as long as the external force is applied to the pump means. However, during that time the maximum amount of beneficial agent that will be expressed to the patient is a full dose. Upon release of the external force, the resilient pump means tends to rebound to its first uncompressed condition, valve means 21 returns to its normally closed position and the dose reservoir is sealed. Thus, as pump means 23 rebounds to its first uncompressed condition, at least a partial vacuum is created in the dose reservoir. The vacuum in the dose reservoir is sufficient to draw beneficial liquid from an external supply source 35 through conduit means 40 and into dose reservoir 30 to replenish beneficial agent that was expressed from the device. Because of the design of the pump means, it rebounds to its first uncompressed condition at a relatively constant rate and, therefore, liquid is likewise drawn from external supply source 35 into dose reservoir 30 at a relatively constant rate.

The dose reservoir 30 is filled in a predetermined time period which will depend on the volume of dose reservoir, the pump means employed and the restriction means, if any, between the beneficial agent supply source 35 and the device 10. It will be appreciated, however, that a patient-controlled analgesic device with a particular pumping rate and dose reservoir volume when manufactured may be used for a variety of dosage requirements by adjusting the concentration of beneficial agent in the supply source to be delivered to the device.

With the device as described, a patient who has depressed head 26 of pump means 23 and has thereby received a dose of beneficial agent may receive another full dose of beneficial agent after the expiration of predetermined refill time period T. While the patient may wait longer than the predetermined time period T if desired, the patient must wait at least the predetermined time period T in order to receive a full dose. However, if the patient depresses pump means 23 sometime before the predetermined time period T required to fill the dose reservoir has expired, the patient will receive only a fraction of the full dose. Because the rebound rate of the pump means is linear over the entire range of completely empty to completely filled, the dose fraction that the patient receives will be equal to the fraction of the refill time period T that has elapsed during the patient's intermittent demand for beneficial agent. For example, if the patient depresses the pump means at a time equal to one-half the predetermined fill time T, the patient will receive only one-half of a full dose volume. Thus no matter how often the patient depresses the pump means, he will never receive more than a single full dose of beneficial agent during the predetermined fill period T.

It will be appreciated that while pump means 23 of the patient-controlled analgesic device 10 of the present invention is capable of drawing beneficial liquid into the dose reservoir 30 at a linear rate, the precise rate at which liquid is drawn may be controlled by suitably restricting the rate at which the beneficial liquid is drawn from the supply source. To that end, either the inlet means itself may be designed to limit its rate of flow, or some other restrictive means within the supply source or between the supply source and the inlet means may be used. In a preferred embodiment of the invention, a conventional administration set is used to control the rate at which beneficial agent is drawn from the supply source. Such administrative sets are available with flow restrictive orifices of a fixed or adjustable design and may be used advantageously in combination with the patient controlled analgesic device of the present invention to control the rate at which beneficial agent is delivered from the supply source.

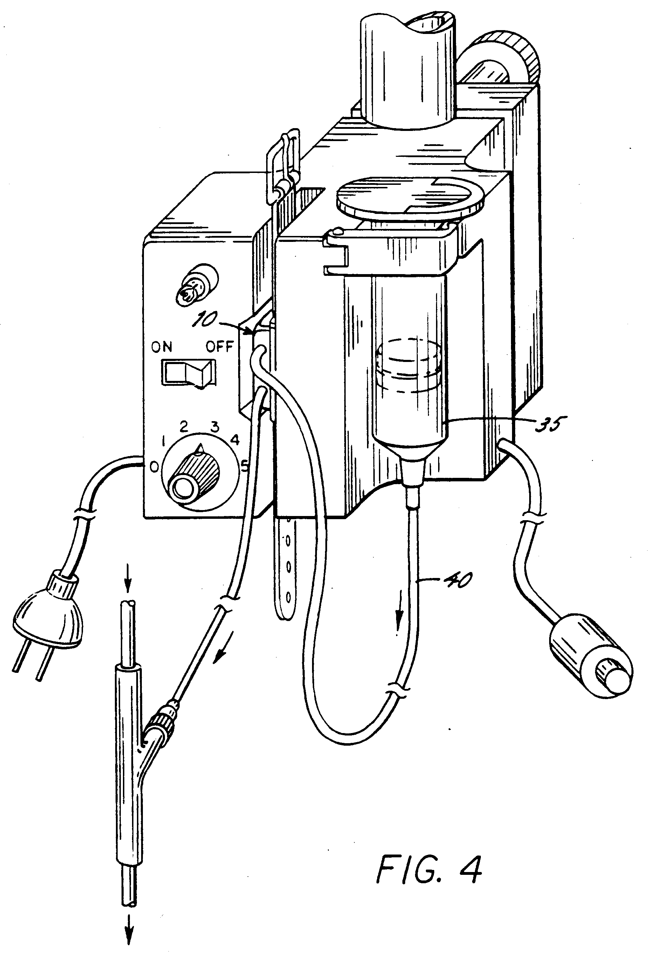

As depicted in FIG. 4, the device 10 of the present invention may be used with automated equipment to deliver a dose of beneficial agent continuously or automatically at timed intervals, particularly when the patient is asleep. The automated equipment is capable of automatically depressing the pump means 23 of the device 10 at preset intervals and may be relatively inexpensive compared to a separate automated pump and power source. It need not be a sophisticated electronic pump because all metering and dosing of beneficial agent will still be done by the device 10, without an external pump. The automated equipment may carry a patient override to permit the patient to override the preset intervals for dose administration. Dosing with such an arrangement is still controlled by the device 10 itself, as previously described, so that patient overdosing is precluded.

The present invention thus provides an accurate, self-driven, low-cost patient controlled analgesic apparatus that is versatile in use. It can be used by ambulatory patients during their waking hours and it can be readily adapted for use by patients at night with relatively inexpensive automated equipment.

## Claims

1. An apparatus (10) for the patient-controlled delivery of a beneficial agent comprising:
(a) a dose reservoir (30) for receiving and storing a dose of beneficial agent;
(b) an inlet means (19) to said dose reservoir for receiving beneficial agent;
(c) an outlet means (20) to said dose reservoir through which beneficial agent in said dose reservoir exits said dose reservoir;
(d) pump means (23) partially defining said dose reservoir (30) operative in response to an external force supplied to said pump means for drawing beneficial agent into said dose reservoir through said inlet means (19) and for discharging the beneficial agent out of said dose reservoir through said outlet means (20),
said apparatus being characterized by said pump means (23) comprising an annular shaped arcuate wall means (25) terminating at a foot (24), said arcuate wall means (25) having a constant wall curvature and wall thickness extending from said foot (24) to a centrally located head means (26), said head means (26) having a rigidity substantially greater than the rigidity of the arcuate wall means (25) to restrict the compression of said pump means (23) substantially to the said arcuate wall means (25), said pump means (23) being compressed in response to an external force applied thereto so as to advance the beneficial agent from said dose reservoir (30) to said outlet means (20), and said pump means (23) returning upon removal of the external force to the uncompressed condition at relatively constant rate,
wherein upon returning to the uncompressed condition the pressure in said dose reservoir (30) is lower relative to the pressure in the external supply (35) so that beneficial agent is drawn into said dose reservoir from the external supply via suction at a relatively constant rate.

2. The apparatus of claim 1, wherein said pump means (23) may be actuated by the patient.

3. The apparatus of claim 2, wherein the resilient material of said pump means (23) prevents delivery of a dose quantity of beneficial agent to the patient in excess of the dose quantity within a preselected time period by returning to the uncompressed condition at a relatively constant rate.

4. The apparatus in accordance with claim 1, further comprising means for automatically compressing the pump means (23) at preset intervals, wherein said pump means (23) is actuated at timed intervals by means for so activating said pump means.

5. The apparatus in accordance with claim 1, wherein said pump means (23) may be repeatedly actuated by the patient, said pump means permitting the delivery of fractional doses of the beneficial agent to the patient at a delivery rate controlled by said pump means so as to prevent exceeding a pre-determined dosage within a pre-determined period of time.

6. The apparatus in accordance with claim 1, including, in combination, an upstream conduit (40) in communication with said inlet means (19) to said dose reservoir (30), said upstream conduit (40) comprising means for controlling the rate of flow of beneficial agent to said dose reservoir (30) in response to actuation of said pump means (23).

7. The apparatus of claim 6, wherein said pump means (23) may be repeatedly actuated by the patient at times less than required for a full dose, said pump means (23) permitting the delivery of fractional doses of the beneficial agent to the patient.

8. The apparatus of claim 7, wherein said pump means (23) prevents delivery of a quantity of beneficial agent to the patient in excess of a dose quantity within a preselected time period.

9. The apparatus of claim 1 or 6, wherein said pump means (23) and said dose reservoir (30) permit the delivery of no greater than a dose quantity of beneficial agent to be delivered to the patient within a preselected time period.

10. The apparatus of claim 1 or 6, including downstream conduit secured at one end to said outlet means (20) and having a distal end adaptable for securement to an intravenous catheter.

11. The apparatus in accordance with claim 1 or 6, wherein said apparatus is free of any electrical power source.

12. The apparatus in accordance with claim 6, said apparatus further comprising means for automatically compressing the pump means at preset intervals, wherein said pump means is actuated at timed intervals by said automatic compressing means.

13. The apparatus in accordance with claim 6, wherein said pump means may be repeatedly actuated by the patient, said pump means permitting the delivery of multiple doses of the beneficial agent to the patient at a controlled delivery rate.

## Patentansprüche

1. Vorrichtung (10) zur patientengesteuerten Abgabe eines wohltuenden (heilsamen) Mittels, umfassend:
(a) ein Dosisreservoir (30) zur Aufnahme und Speicherung einer Dosis wohltuenden Mittels;
(b) einen Einlaß (19) zu dem Dosisreservoir für die Aufnahme wohltuenden Mittels;
(c) einen Auslaß (20) zu dem Dosisreservoir, durch welchen wohltuendes Mittel in dem Dosisreservoir aus dem Dosisreservoir austritt;
(d) eine das Dosisreservoir (30) teilweise begrenzende Pumpeinrichtung (23), die in Abhängigkeit einer auf die Pumpeinrichtung ausgeübten äußeren Kraft wirksam ist, um wohltuendes Mittel durch den Einlaß (19) in das Dosisreservoir einzuziehen und das wohltuende Mittel durch den Auslaß (20) aus dem Dosisreservoir auszugeben,
wobei die Vorrichtung dadurch gekennzeichnet ist, daß die Pumpeinrichtung (23) eine bogenförmige Ringwand (25) aufweist, die in einem Fuß (24) endet, daß die Ringwand (25), die eine konstante Wandkrümmung und eine konstante Wandstärke besitzt, sich von dem Fuß (24) zu einem zentral gelegenen Kopf (26) erstreckt, der eine Steifigkeit besitzt, die wesentlich größer ist als die Steifigkeit der bogenförmigen Wand (25), um die Kompression der Pumpeinrichtung (23) im wesentlichen auf die bogenförmige Wand (25) zu begrenzen, daß die Pumpeinrichtung (23) in Abhängigkeit einer auf sie ausgeübten äußeren Kraft komprimiert wird, um das wohltuende Mittel aus dem Dosisreservoir (30) zu dem Auslaß (20) vorzutreiben,
und daß die Pumpeinrichtung (23) bei Fortfall der äußeren Kraft mit verhältnismäßig konstanter Geschwindigkeit in den nicht komprimierten Zustand zurückkehrt, wobei beim Zurückkehren in den nicht komprimierten Zustand der Druck in dem Dosisreservoir (30) geringer ist gegenüber dem Druck in dem externen Vorrat (35), sodaß wohltuendes Mittel aus dem externen Vorrat durch Saugwirkung mit einer verhältnismäßig konstanten Strömungsrate in das Dosisreservoir gezogen wird.

2. Vorrichtung nach Anspruch 1, dadurch gekennzeichnet, daß die Pumpeinrichtung (23) von dem Patienten betätigt werden kann.

3. Vorrichtung nach Anspruch 2, dadurch gekennzeichnet, daß das elastische Material der Pumpeinrichtung (23) durch Zurückkehren in den nicht komprimierten Zustand mit einer verhältnismäßig konstanten Geschwindigkeit die Abgabe einer die Dosismenge innerhalb einer vorgewählten Zeitspanne übersteigenden Dosismenge an wohltuendem Mittel an den Patienten verhindert.

4. Vorrichtung nach Anspruch 1, weiters umfassend Mittel zum automatischen Komprimieren der Pumpeinrichtung (23) in vorbestimmten Intervallen, dadurch gekennzeichnet, daß die Pumpeinrichtung (23) in zeitlich festgelegten Intervallen durch Mittel betätigt wird, um so die Pumpeinrichtung in Betrieb zu setzen.

5. Vorrichtung nach Anspruch 1, dadurch gekennzeichnet, daß die Pumpeinrichtung (23) durch den Patienten wiederholt betätigt werden kann, wobei die Pumpeinrichtung die Abgabe von fraktionären Dosen des wohltuenden Mittels an den Patienten mit einer von der Pumpeinrichtung gesteuerten Abgaberate erlaubt, um zu verhindern, daß eine vorbestimmte Dosierung innerhalb einer vorbestimmten Zeitspanne überschritten wird.

6. Vorrichtung nach Anspruch 1, enthaltend in Kombination eine stromauf gelegene Leitung (40) in Verbindung mit dem Einlaß (19), wobei die stromauf gelegene Leitung (40) Mittel zur Steuerung der Strömungsrate wohltuenden Mittels zu dem Dosisreservoir (30) in Abhängigkeit von der Betätigung der Pumpeinrichtung (23) umfaßt.

7. Vorrichtung nach Anspruch 6, dadurch gekennzeichnet, daß die Pumpeinrichtung (23) von dem Patienten wiederholt zu Zeiten, die geringer sind als für eine volle Dosis erforderlich, betätigt werden kann, wobei die Pumpeinrichtung (23) die Abgabe fraktionärer Dosen des wohltuenden Mittels an den Patienten zuläßt.

8. Vorrichtung nach Anspruch 7, dadurch gekennzeichnet, daß die Pumpeinrichtung (23) die Abgabe einer Menge an wohltuendem Mittel an den Patienten, welche eine Dosismenge innerhalb einer vorgewählten Zeitspanne übersteigt, verhindert.

9. Vorrichtung nach Anspruch 1 oder 6, dadurch gekennzeichnet, daß die Pumpeinrichtung (23) und das Dosisreservoir (30) die Abgabe einer Dosismenge an wohltuendem Mittel erlauben, die nicht größer ist als die innerhalb einer vorgewählten Zeitspanne an dem Patienten abzugebende Dosismenge.

10. Vorrichtung nach Anspruch 1 oder 6, enthaltend eine stromab gelegene Leitung, die an einem Ende an dem Auslaß (20) befestigt ist und ein für die Befestigung an einem intravenösen Katheter adaptierbares distales Ende besitzt.

11. Vorrichtung nach Anspruch 1 oder 6, dadurch gekennzeichnet, daß die Vorrichtung frei jeder elektrischen Energiequelle ist.

12. Vorrichtung nach Anspruch 6, weiters umfassend Mittel zum automatischen Komprimieren der Pumpeinrichtung in vorbestimmten Intervallen, dadurch gekennzeichnet, daß die Pumpeinrichtung von den automatischen Komprimiermitteln in zeitgesteuerten Intervallen betätigt wird.

13. Vorrichtung nach Anspruch 6, dadurch gekennzeichnet, daß die Pumpeinrichtung von dem Patienten wiederholt betätigt werden kann, wobei die Pumpeinrichtung die Abgabe von Mehrfachdosen des wohltuenden Mittels an den Patienten mit einer gesteuerten Abgaberate erlaubt.

## Revendications

1. Appareil (10) pour la fourniture contrôlée par un patient d'un produit bénéfique, comprenant :
(a) une dose-réservoir (30) pour recevoir et stocker une dose de produit bénéfique ;
(d) une entrée (19) vers ladite dose-réservoir pour recevoir du produit bénéfique ;
(c) une sortie (20) vers ladite dose-réservoir, à travers laquelle ledit produit bénéfique dans ladite dose-réservoir sort de ladite dose-réservoir ;
(d) des moyens de pompage (23) qui définissent partiellement ladite dose-réservoir (30) et capables de fonctionner en réponse à une force externe appliquée auxdits moyens de pompage pour aspirer des produits bénéfiques dans ladite dose-réservoir via ladite entrée (19) et pour décharger le produit bénéfique hors de ladite dose-réservoir à travers ladite sortie (20),
ledit appareil étant caractérisé en ce que lesdits moyens de pompage (23) comprennent une paroi annulaire cintrée (25) qui se termine par un pied (24), ladite paroi cintrée (25) ayant une courbure de paroi et une épaisseur de paroi constantes qui s'étendent depuis ledit pied (24) jusqu'à une tête située au centre (26), ladite tête (26) ayant une rigidité sensiblement plus élevée que la rigidité de la paroi cintrée (25) pour restreindre la compression desdits moyens de pompage (23) sensiblement à ladite paroi cintrée (25), lesdits moyens de pompage (23) étant comprimés en réponse à une force externe qui leur est appliquée de manière à faire avancer le produit bénéfique depuis ladite dose-réservoir (30) vers ladite sortie (20), et lesdits moyens de pompage (23) retournant, lorsqu'on supprime la force externe, à la condition non-comprimée à une vitesse relativement constante,
dans lequel, lors du retour à la condition non-comprimée, la pression dans ladite dose-réservoir (30) est plus faible que la pression dans l'alimentation externe (35) de sorte que le produit bénéfique est aspiré dans ladite dose-réservoir depuis l'alimentation externe par succion à une vitesse relativement constante.

2. Appareil selon la revendication 1, dans lequel lesdits moyens de pompage (23) peuvent être actionnés par le patient.

3. Appareil selon la revendication 2, dans lequel le matériau élastique desdits moyens de pompage (23) empêche la fourniture d'une quantité de dose de produit bénéfique au patient en excès de la quantité de dose à l'intérieur d'une période de temps prédéterminée en retournant à la condition non-comprimée à une vitesse relativement constante.

4. Appareil selon la revendication 1, comprenant en outre des moyens pour comprimer automatiquement les moyens de pompage (23) à des intervalles présélectionnés, dans lequel lesdits moyens de pompage (23) sont actionnés à des intervalles temporisés par des moyens destinés à activer ainsi lesdits moyens de pompage.

5. Appareil selon la revendication 1, dans lequel lesdits moyens de pompage (23) peuvent être actionnés à répétition par le patient, lesdits moyens de pompage permettant la fourniture de dose fractionnaires du produit bénéfique au patient à une vitesse de fourniture contrôlée par lesdits moyens de pompage, de manière à empêcher le dépassement d'un dosage prédéterminé dans une période de temps prédéterminée.

6. Appareil selon la revendication 1, comprenant, en combinaison, un conduit amont (40) en communication avec ladite entrée (19) vers ladite dose-réservoir (30), ledit conduit amont (40) comprenant des moyens pour commander le débit de produit bénéfique vers ladite dose-réservoir (30) en réponse à l'actionnement desdits moyens de pompage (23).

7. Appareil selon la revendication 6, dans lequel lesdits moyens de pompage (23) peuvent être actionnés à répétition par le patient à des périodes inférieures à celle requise pour une dose entière, lesdits moyens de pompage (23) permettant la fourniture de doses fractionnaires du produit bénéfique au patient.

8. Appareil selon la revendication 7, dans lequel lesdits moyens de pompage (23) empêchent la fourniture d'une quantité de produit bénéfique au patient en excès d'une quantité de dose à l'intérieur d'une période de temps présélectionnée.

9. Appareil selon l'une ou l'autre des revendications 1 et 6, dans lequel lesdits moyens de pompage (23) et ladite dose-réservoir (30) permettent la fourniture d'une quantité de produit bénéfique qui n'est pas supérieure à une dose à administrer au patient à l'intérieur d'une période de temps présélectionnée.

10. Appareil selon l'une ou l'autre des revendications 1 et 6, comprenant un conduit aval fixé à une extrémité à ladite sortie (20) et présentant une extrémité distale adaptée pour être attachée à un cathéter intraveineux.

11. Appareil selon l'une ou l'autre des revendications 1 et 6, dans lequel ledit appareil est dépourvu d'une quelconque source de puissance électrique.

12. Appareil selon la revendication 6, ledit appareil comprenant en outre des moyens pour comprimer automatiquement les moyens de pompage à des intervalles présélectionnés, dans lequel lesdits moyens de pompage sont actionnés à des intervalles temporisés par lesdits moyens de compression automatiques.

13. Appareil selon la revendication 6, dans lequel lesdits moyens de pompage peuvent être actionnés à répétition par le patient, lesdits moyens de pompage permettant la fourniture de doses multiples du produit bénéfique au patient à une vitesse de fourniture contrôlée.
